# EUROPÄISCHE PATENTSCHRIFT

(11) **EP 0 878 628 B1**
(45) Veröffentlichungstag und Bekanntmachung des Hinweises auf die Patenterteilung: **12.04.2000**
(21) Anmeldenummer: 98108225.8
(22) Anmeldetag: 06.05.1998
(51) Int. Cl.: F15B 3/00, A61M 1/36, B29C 65/08

(54) **Druckübertragungsvorrichtung**
Pressure transmission device
Dispositif de transfert de pression

(30) Priorität: 15.05.1997 DE 29708673 U
(43) Veröffentlichungstag der Anmeldung: 18.11.1998
(73) Patentinhaber: Filtertek B.V., Limerick (IE)
(72) Erfinder: Myers, Jan Willem Marinus, 5913 TP Venlo (NL)
(74) Vertreter: Brose, D. Karl

(56) Entgegenhaltungen:
- EP-A- 0 652 018
- DE-U- 29 708 673
- US-A- 3 966 520
- US-A- 4 459 139

## Beschreibung

Die vorliegende Erfindung betrifft eine Vorrichtung zur Übertragung des Drucks im sterilen Bereich eines medizinischen Geräts auf ein nicht steriles Druckmeßgerät nach dem Oberbegriff des Anspruchs 1.

Solche Druckübertragungsvorrichtungen werden beispielsweise bei Hämodialysemaschinen, Herz-Lungen-Maschinen und ähnlichen medizinischen Geräten benötigt, bei denen eine sehr genaue Überwachung der herrschenden Drücke in einem sterilen Schlauchsystem erforderlich ist, gleichzeitig aber eine Sterilisierung der entsprechenden Druckmeßgeräte nicht praktikabel ist.

Diese Druckübertragungsvorrichtungen müssen absolut dicht und leckfrei sein, damit nicht unsterile Luft in den sterilen Bereich des medizinischen Geräts gelangt, oder der Druckmeßwert verfälscht wird. Außerdem müssen diese Druckübertragungsvorrichtungen an der nicht sterilen Seite einen sogenannten "Luer-Lock"-Anschluß aus einem Polyester/Polyäther-Copolymer aufweisen, da nur mit einem solchen Anschluß eine leicht trennbare und trotzdem absolut leckfreie Verbindung mit den entsprechenden Edelstahl-Anschlußelementen der Druckmeßgeräte in dem medizinischen Gerät ermöglicht wird.

Die bisher am Markt befindlichen Druckübertragungsvorrichtungen dieser Art, wie sie beispielsweise in der EP 0 652 018 A beschrieben sind, waren dergestalt aufgebaut, daß das Gehäuse aus einer sterilseitigen Hälfte aus Polycarbonat mit einem Schlauchanschluß und einer nicht sterilseitigen Hälfte aufgebaut war, und zwischen diesen Hälften eine Filter-Membran aus einem permeablen Material zur Druckübertragung bei gleichzeitiger völliger Dichtheit angeordnet war. Die entsprechende nichtsterilseitige "Luer-Lock"-Anschlußvorrichtung mußte dabei bei der Herstellung der nichtsterilseitigen Hälfte des Gehäuses in einem separaten Spritzgießvorgang zugespritzt werden. Die Fachwelt glaubte, von dieser Herstellungsweise nicht abweichen zu können, da in der Fachwelt das Vorurteil bestand, daß eine absolut dichte und leckfreie Verbindung zwischen einem Teil aus Polyester/Polyäther-Copolymer und einem Teil aus Polycarbonat nur durch das aufwendige Zuspritzverfahren und keinesfalls durch Ultraschallschweißen hergestellt werden könnte. Außerdem war man der Meinung, daß eine vollständig dichte Befestigung des Vlieses mit Polytetrafluoräthylenfolie nur zwischen zwei Gehäusehälften aus Polycarbonat möglich wäre, da das Polyester/Polyäther-Copolymer dazu zu weich wäre.

Ausgehend von diesem Stand der Technik ist es Aufgabe der vorliegenden Erfindung, eine Druckübertragungsvorrichtung der vorgenannten Art zu schaffen, die bei gleicher Qualität (Dichtigkeit und Leckfreiheit) wesentlich einfacher und preiswerter herzustellen ist.

Erfindungsgemäß wird die Aufgabe durch die Merkmale des Anspruchs 1 gelöst.

Entgegen dem in der Fachwelt verbreiteten Vorurteil hat die vorliegende Erfindung die Erkenntnis zum Gegenstand, daß sehr wohl eine hinreichend dichte und leckfreie Ultraschallschweißverbindung zwischen einer Gehäusehälfte aus Polyester/Polyäther-Copolymer und einer Gehäusehälfte aus Polycarbonat herstellbar ist.

Es hat sich dabei als besonders vorteilhaft erwiesen, das Vlies, welches die Polytetrafluoräthylenfolie stützt, aus Polyester herzustellen. Dies hat im wesentlichen den Vorteil, daß das Vlies beim Ultraschallschweißvorgang zwischen den Gehäusehälften mitverschweißt wird, wodurch eine erheblich bessere Befestigung und Abdichtung des Vlieses erzielt wird.

Weiter ist es besonders bevorzugt, daß die nichtsterilseitige Hälfte des Gehäuses einen dünnen umlaufenden Vorsprung aufweist, der ein Stück über die sterilseitige Hälfte des Gehäuses übergreift und dort die Ultraschallverschweißungszone bildet. Auf diese Weise läßt sich die Ultraschallverschweißung besonders einfach, sicher und kostengünstig durchführen.

Weiter ist es besonders bevorzugt, innerhalb des Vorsprunges eine umlaufende Nut in der nichtsterilseitigen Hälfte vorzusehen, in die ein Vorsprung der sterilseitigen Hälfte eingreift. Auf diese Weise wird die Ultraschallverschweißungszone verbreitert und die Dichtigkeit und Festigkeit der Verbindung der beiden Gehäusehälften weiter gesteigert.

Besonders bevorzugt ist es dabei, daß der Vorsprung der sterilseitigen Hälfte an seiner Außenseite eine auf die nichtsterilseitige Hälfte zulaufende Anfasung aufweist. Hierdurch entsteht ein schmaler, keilförmiger, umlaufender Hohlraum am nichtsterilseitigen Ende der Verschweißungszone, in den das bei der Ultraschallverschweißung aufgeweichte Material ausweichen kann, wodurch die Qualität der Verschweißung weiter gesteigert wird.

Ebenso ist es besonders bevorzugt, die Nut an ihrer Innenseite abzuschrägen. Dadurch wird der Zusammenbau der beiden Gehäusehälften vereinfacht und zusätzlicher Raum für das Ausweichen von durch das Ultraschallschweißen erweichten Material geschaffen.

Die vorliegende Erfindung wird im folgenden anhand der beigefügten Zeichnung eines Ausführungsbeispiels näher erläutert. Es zeigen:
**Figur 1** eine teilweise Schnittdarstellung durch eine vollständige erfindungsgemäße Druckübertragungsvorrichtung;

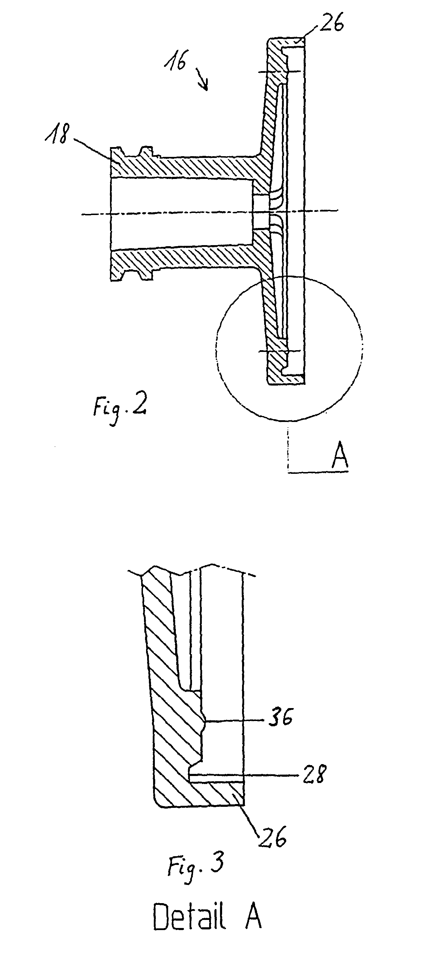

**Figur 2** eine vollständige Schnittdarstellung der nichtsterilseitigen Gehäusehälfte der erfindungsgemäßen Druckübertragungsvorrichtung nach Fig. 1;
**Figur 3** eine vergrößerte Darstellung des Details A der Figur 2;

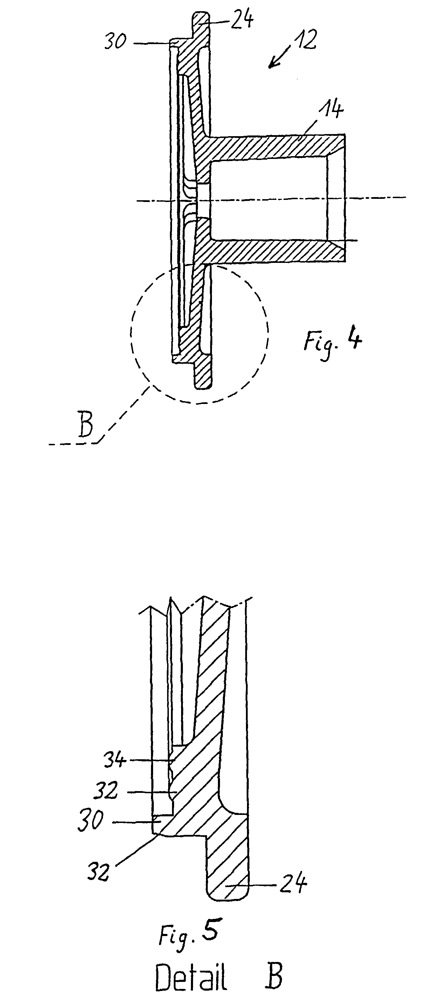

**Figur 4** eine vollständige Schnittdarstellung der sterilseitigen Gehäusehälfte der erfindungsgemäßen Druckübertragungsvorrichtung gem. Fig. 1;
**Figur 5** eine vergrößerte Darstellung des Details B der Figur 4.

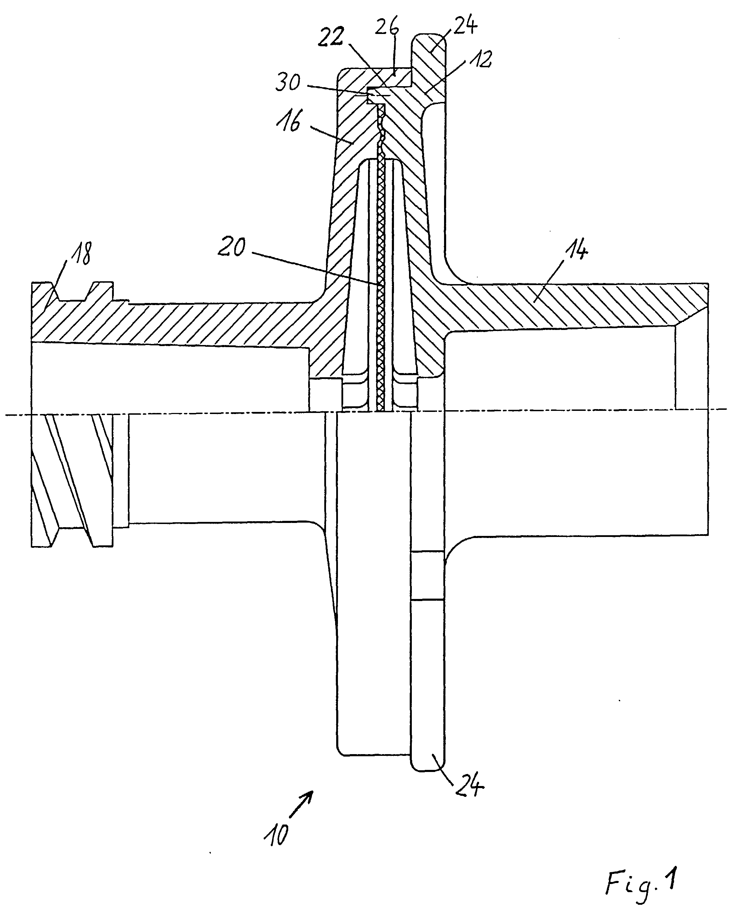

Wie in Figur 1 dargestellt, besteht das Gehäuse 10 einer erfindungsgemäßen Druckübertragungsvorrichtung aus einer sterilseitigen Gehäusehälfte 12 mit einem Schlauchanschluß 14, die aus Polycarbonat besteht, und einer nichtsterilseitigen Gehäusehälfte 16 mit einem "Luer-Lock"-Anschluß 18, die erfindungsgemäß vollständig aus Polyester/Polyäther-Copolymer besteht. Zwischen den beiden Gehäusehälften 12 und 16 ist ein Vlies 20 aus Polyester eingepresst und durch das Ultraschallverschweißen der beiden Gehäusehälften 12 und 16 mitverschweißt. Dieses Vlies 20 trägt eine dünne Polytetrafluoräthylenfolie, die als permeable Filter-Membran die Filterfunktion zwischen dem sterilen und dem nichtsterilen Bereich bildet, um eine entsprechende Luftdruckübertragung zu ermöglichen. Die Ultraschallverschweißung zwischen den beiden Gehäusehälften 12 und 16 erfolgt dabei in dem mit 22 gekennzeichneten Bereich.

Die beiden Gehäusehälften 12 und 16 sind dabei jeweils zylindrisch gestaltet, so daß die ganze Konstruktion rotationssymmetrisch ist. Die Verbindung zwischen den beiden Gehäusehälften 12 und 16 wird dabei im einzelnen folgendermaßen erzielt:

Die sterilseitige Gehäusehälfte 12 weist einen außen umlaufenden Ring 24 auf. Dieser Ring 24 dient als Anschlag für einen dünnen, umlaufenden Vorsprung 26, der sich von der nichtsterilseitigen Gehäusehälfte 16 aus in Richtung auf die sterilseitige Gehäusehälfte 12 hin erstreckt. Innerhalb des Vorsprunges 26 verläuft direkt an diesen anschließend eine umlaufende Nut 28 in der nichtsterilseitigen Gehäusehälfte 16. In diese Nut 28 greift ein Vorsprung 30 der sterilseitigen Gehäusehälfte 12 ein. Dieser Vorsprung 30 weist an seinem der nichtsterilseitigen Hälfte 12 zugekehrten Ende eine Abschrägung 32 an seiner Außenseite auf, so daß die Dicke des Vorsprungs 30 auf die nichtsterilseitige Hälfte 12 zu abnimmt.

Die Ultraschallschweißverbindung erfolgt in dem mit 22 gekennzeichneten Bereich von außen und umlaufend. Die Abschrägung 32 dient dazu, einen Hohlraum in der Nut 28 zu schaffen, in den das durch die Ultraschallverschweißung aufgeschmolzene Material ausweichen kann. Die Innenseite der Nut 28 in der nichtsterilseitigen Gehäusehälfte 16 ist ebenfalls abgeschrägt, um eine leichtere Montage sicherzustellen und zusätzlich weiteren Ausweichraum für beim Ultraschallschweißen aufgeschmolzenes Material zur Verfügung zu stellen.

Innerhalb des Vorsprungs 30 bzw. der Nut 28 ist das Vlies 20 zwischen den beiden Gehäusehälften 12 und 16 eingepresst, welches die Polytetrafluoräthylenfolie zur Abdichtung des sterilen vom nichtsterilen Bereich trägt. Dieses Vlies 20 besteht vorzugsweise aus Polyester. Zur besseren Befestigung und Abdichtung des Vlieses 20 zwischen den beiden Gehäusehälften 12 und 16 sind auf der sterilseitigen Gehäusehälfte 12 zwei leicht vorstehende konzentrische Ringe 32 und 34 vorgesehen. In dem Bereich zwischen diesen beiden Ringen 32 und 34 erstreckt sich von der gegenüberliegenden Seite ein weiterer einzelner umlaufender Ring 36 auf der nichtsterilseitigen Gehäusehälfte 16.

Zum Zusammenbau der Druckübertragungsvorrichtung wird ein Vlies 20 mit Polytetrafluoräthylenfolie in eine sterilseitige Gehäusehälfte eingelegt, die nichtsterilseitige Gehäusehälfte aufgesteckt und die beiden Hälften durch Ultraschallschweißen rundum dicht verbunden. Durch dieses Herstellungsverfahren kann die Druckübertragungsvorrichtung erheblich preisgünstiger hergestellt werden, als dies gemäß dem Stand der Technik möglich ist.

## Patentansprüche

1. Vorrichtung zur Übertragung des Druckes im sterilen Bereich eines medizinischen Geräts auf ein nichtsteriles Druckmeßgerät mit einem nichtsterilen Anschluß (18) aus Polyester/Polyäther-Copolymer und einem sterilseitigen Anschluß (14) aus Polycarbonat, wobei zwischen den beiden Anschlüssen (14, 18) eine Filter-Membran aus permeablem Material flüssigkeitsdicht zwischen zwei Hälften (12, 16) eines Gehäuses (10) angeordnet ist, wobei die sterilseitige Hälfte (12) des Gehäuses (10) aus Polycarbonat besteht, **dadurch gekennzeichnet**, daß die Filter-Membran aus Polytetrafluoräthylen besteht und hydrophob ist, daß die Filter-Membran von einem Vlies (20) gestützt wird und daß die nichtsterilseitige Hälfte (16) des Gehäuses vollständig aus Polyester/Polyäther-Copolymer besteht und die beiden Hälften (12, 16) durch Ultraschallschweißen verbunden sind.

2. Vorrichtung nach Anspruch 1, **dadurch gekennzeichnet**, daß das Vlies (20) aus Polyester besteht.

3. Vorrichtung nach Anspruch 1 oder 2, **dadurch gekennzeichnet**, daß die nichtsterilseitige Hälfte (16) des Gehäuses einen dünnen umlaufenden Vorsprung (26) aufweist, der ein Stück über die sterilseitige Hälfte (12) des Gehäuses übergreift und dort die Ultraschallverschweißungszone (22) bildet.

4. Vorrichtung nach Anspruch 3, **dadurch gekennzeichnet**, daß innerhalb des Vorsprunges (26) eine umlaufende Nut (28) in der nichtsterilseitigen Hälfte (16) vorgesehen ist, in die ein Vorsprung (30) der sterilseitigen Hälfte (12) eingreift.

5. Vorrichtung nach Anspruch 4, **dadurch gekennzeichnet**, daß der Vorsprung (30) der sterilseitigen Hälfte (12) an seiner Außenseite eine auf die nichtsterilseitige Hälfte (16) zulaufende Anfasung (32) aufweist.

6. Vorrichtung nach Anspruch 4 oder 5, **dadurch gekennzeichnet**, daß die Nut (28) an ihrer Innenseite abgeschärft ist.

## Claims

1. Apparatus for transmitting the pressure in the sterile section of a medical appliance to a non-sterile pressure gauge, having a non-sterile connector (18) of polyester/polyether-copolymer and a connector on the sterile side of polycarbonate, wherein between the two connectors (14, 18) a filter diaphragm of a permeable material is positioned in a fluid tight manner between two halves (12, 16) of a housing (10), wherein the half (12) on the sterile side of the housing (10) is consisting of polycarbonate, characterized in that the filter diaphragm is consisting of polytetrafluorethylene and is hydrophobic, that the filter diaphragm is supported by a fleece (20) and, in that the half (16) of the non-sterile side of the housing completely is consisting of polyester/polyether-copolymer and the two halves (12, 16) being connected by ultrasonic welding.

2. Apparatus according to claim 1, characterized in that the fleece (20) is consisting of polyester.

3. Apparatus according to claim 1 or 2, characterized in that the half (16) of the non-sterile side of the housing is having a thin circumferential projection (26) overlapping for a distance the half (12) of the sterile side of the housing and there is forming the ultrasonic welding zone (22).

4. Apparatus according to claim 3, characterized in that within the projection (26) a circumferential groove (28) is provided in the half (16) of the non-sterile side into which a projection (30) of the half (12) of the sterile side is projecting.

5. Apparatus according to claim 4, characterized in that the projection (30) of the half (12) of the sterile side on its exterior is having a bevel (32) in the direction of the half (16) of the non-sterile side.

6. Apparatus according to claim 4 or 5, characterized in that the groove (28) is bevelled on its interior side.

## Revendications

1. Dispositif de transmission de la pression dans la zone stérile d'un appareil médical à un appareil de mesure de pression non stérile comportant un raccord non stérile (18) en copolymère de polyester/polyéther ainsi qu'un raccord côté stérile (14) en polycarbonate, entre les deux raccords (14, 18) une membrane de filtre en matière perméable étant disposée de façon étanche aux liquides, entre deux moitiés (12, 16) d'un boîtier (10), la moitié côté stérile (12) du boîtier (10) étant en polycarbonate, caractérisé en ce que la membrane de filtre est en polytétrafluoréthylène et hydrophobe, en ce que la membrane de filtre est soutenue par un non-tissé (20) et en ce que la moitié côté non stérile (16) du boîtier est totalement en copolymère de polyester/polyéther et les deux moitiés (12, 16) sont assemblées par soudage aux ultrasons.

2. Dispositif selon la revendication 1, caractérisé en ce que le non-tissé (20) est en polyester.

3. Dispositif selon la revendication 1 ou 2, caractérisé en ce que la moitié côté non stérile (16) du boîtier présente une mince saillie (26) périphérique qui passe sur une partie de la moitié côté stérile (12) du boîtier et y forme la zone (22) de soudure aux ultrasons.

4. Dispositif selon la revendication 3, caractérisé en ce qu'à l'intérieur de la saillie (26) est prévue une rainure périphérique (28) dans la moitié côté non stérile (16), dans laquelle s'engage une saillie (30) de la moitié côté stérile (12).

5. Dispositif selon la revendication 4, caractérisé en ce que la saillie (30) de la moitié côté stérile (12) présente, sur son côté extérieur, un chanfrein (32) dirigé vers la moitié côté non stérile (16).

6. Dispositif selon la revendication 4 ou 5, caractérisé en ce que la rainure (28) est chanfreinée sur son côté intérieur.
